# EUROPÄISCHE PATENTSCHRIFT

(11) **EP 0 767 625 B1**
(45) Veröffentlichungstag und Bekanntmachung des Hinweises auf die Patenterteilung: **24.03.1999**
(21) Anmeldenummer: 95919412.7
(22) Anmeldetag: 05.05.1995
(51) Int. Cl.: A61B 17/28

(54) **CHIRURGISCHES INSTRUMENT**
SURGICAL INSTRUMENT
INSTRUMENT CHIRURGICAL

(30) Priorität: 28.06.1994 DE 4422621
(43) Veröffentlichungstag der Anmeldung: 16.04.1997
(73) Patentinhaber: Aesculap AG & Co. KG, 78532 Tuttlingen (DE)
(72) Erfinder: KIENZLE, Karl-Ernst, D-78532 Tuttlingen (DE); MAYENBERGER, Rupert, D-78239 Rielasingen (DE); NESPER, Markus, D-78532 Tuttlingen (DE); WEISSHAUPT, Dieter, D-78194 Immendingen (DE)
(74) Vertreter: Böhme, Ulrich, Dr. Dipl.-Phys.
(86) Internationale Anmeldenummer: EP9501718
(87) Internationale Veröffentlichungsnummer: WO9600526

(56) Entgegenhaltungen:
- EP-A- 0 546 767
- WO-A-93/22980
- WO-A-93/25267
- DE-A- 2 506 471
- DE-A- 3 119 550
- DE-A- 4 223 162
- US-A- 3 750 652

## Beschreibung

Die Vorliegende Erfindung betrifft ein chirurgisches Instrument mit den Merkmalen des Oberbegriffs des vorliegenden Anspruchs 1.

Ein chirurgisches Instrument mit einem Schaft, an dessen Ende Werkzeuge verschwenkbar gelagert sind, die zwischen einer Öffnungs- und einer Schließstellung von um eine Drehachse verschwenkbar gelagerten Schwenkelementen verschwenkt werden können, ist aus der deutschen Offenlegungsschrift DE 43 12 284 A1 bekannt. Die Schwenkelemente lassen sich mit Hilfe eines Betätigungsglieds um die Drehachse verschwenken, wobei das Betätigungsglied von einem den Werkzeugen gegenüberliegenden Ende des Schafts aus betätigbar ist und zwei Arme umfaßt, die jeweils an einem ersten Anlenkpunkt gelenkig mit einem Schwenkelement verbunden sind. Dieses bekannte Instrument ist mit Werkzeugen aus Kunststoff versehen, die eine hohe Biegsamkeit aufweisen und deutlich nachgeben, wenn sie auf einen Widerstand stoßen.

In der deutschen Offenlegungsschrift DE 25 06 471 wird vorgeschlagen, Schwenkelement und Arm derart zueinander auszurichten, daß der Arm in der öffnungsstellung des Werkzeugs schräg zu einer senkrecht zu der Drehachse durch den ersten Anlenkpunkt verlaufenden Achse des Schwenkelements und in der Schließstellung des Werkzeugs im wesentlichen senkrecht zu der Achse steht.

Die aus der deutschen Offenlegungsschrift DE 25 06 471 bekannte Ausgestaltung hat allerdings den Nachteil, daß zum Schließen des Werkzeugs zum einen das innerhalb des Schafts verschiebliche Betätigungselement verschoben werden muß und daß zum anderen zusätzlich eine den Schaft umgebende Führungshülse verschoben werden muß. Nur auf diese Weise ist es möglich, einen großen Winkelabstand zwischen den Lagen des Schwenkelements in der Öffnungs- und der Schließstellung zu erzielen.

Es ist daher Aufgabe der vorliegenden Erfindung, ein chirurgisches Instrument der gattungsgemäßen Art so auszubilden, daß sich bei einfacher Handhabung des chirurgischen Instruments ein besonders großer Winkelabstand zwischen den Lagen des Schwenkelements in der Öffnungs- und der Schließstellung erzielen läßt.

Diese Aufgabe wird bei einem chirurgischen Instrument der eingangs beschriebenen Art erfindungsgemäß dadurch gelöst, daß die Länge des Arms im wesentlichen der lichten Weite des Schafts entspricht.

Diese Ausgestaltung erlaubt es, mittels einer längs des Arms auf das Schwenkelement wirkenden Kraft ein auf das Schwenkelement wirkendes Drehmoment zu erzeugen, das beim Übergang von der Öffnungs- zur Schließstellung anwächst und in der Schließstellung maximal ist. Infolgedessen wachsen auch das auf das Werkzeug wirkende Drehmoment und die Schließkraft beim Übergang von der Öffnungs- zur Schließstellung an. Die zur plastischen verformung eines Gegenstands erforderlichen Verformungskräfte steigen in der Regel ebenfalls beim Übergang von der Öffnungs- in die Schließstellung an. Diese Konstruktion stellt also die günstigsten Hebelverhältnisse und somit das bei vorgegebener längs des Arms übertragener Kraft maximale auf das Schwenkelement wirkende Drehmoment gerade dann zur Verfügung, wenn es benötigt wird, nämlich in der Schließstellung des Werkzeugs.

Der Arm steht in der Öffnungsstellung des Werkzeugs schräg zur Schaftachse und in der Schließstellung des Werkzeugs nahezu senkrecht zur Schaftachse. Dadurch nimmt bei konstanter Verschiebungsgeschwindigkeit des Übertragungsglieds die Winkelgeschwindigkeit des Schwenkelements und damit des Werkzeugs bei Annäherung an die Schließstellung ab, so daß die Position des Werkzeugs in der Nähe der Schließstellung besonders fein eingestellt werden kann.

Da der Arm in der Schließstellung des Werkzeugs nahezu senkrecht zur Schaftachse steht und die Länge des Arms nahezu der lichten Weite des Schafts entspricht, kann durch eine Verschiebung des zweiten Anlenkpunkts parallel zu der Schaftachse der erste Anlenkpunkt quer zur Schaftachse von einer Lage nahe einer Innenwand des Schafts bis zu einer Lage nahe einer dieser gegenüberliegenden Innenwand des Schafts bewegt werden. Dadurch gibt sich ein besonders großer Winkelabstand zwischen den Lagen des Schwenkelements in der Öffnungs- und der Schließstellung.

Bei einer bevorzugten Ausführungsform ist vorgesehen, daß der Arm mit seinem freien Ende an einem zweiten Anlenkpunkt gelenkig mit einem längs der Schaftachse verschiebbaren Übertragungsglied verbunden ist. Damit ergibt sich eine sehr einfache Umsetzung einer direkt längs des Schafts übertragbaren Schub- und Zugbewegung in eine Drehbewegung des Schwenkelements. Die durch den Arm auf das Schwenkelement wirkende Kraft wirkt dabei längs einer durch den ersten und den zweiten Anlenkpunkt des Arms verlaufenden Armachse.

Bei allen Ausführungsformen des erfindungsgemäßen chirurgischen Instruments ist es von Vorteil, wenn der radiale Abstand des ersten Anlenkpunkts von der Drehachse wesentlich größer ist als die lichte Weite des Schafts. Dadurch ergibt sich ein langer Hebelarm für die längs des Arms an dem Schwenkelement angreifende Kraft.

Über die Art der Kopplung zwischen dem Schwenkelement und dem Werkzeug wurden bisher noch keine näheren Angaben gemacht.

Das Schwenkelement kann starr mit dem Werkzeug verbunden sein. Werkzeug und Schwenkelement werden in diesem Fall um eine gemeinsame Drehachse verschwenkt. Dieser Aufbau hat den Vorteil großer Einfachheit und geringer Reibungsverluste, da auf zusätzliche Getriebemittel verzichtet wird.

Das Schwenkelement kann jedoch auch über Getriebemittel mit dem Werkzeug verbunden sein, wenn beispielsweise die Winkelgeschwindigkeit des Werkzeugs gegenüber derjenigen des Schwenkelements unter-/übersetzt werden oder die Drehrichtung des Werkzeugs gegenüber derjenigen des Schwenkelements geändert werden soll.

Vorteilhafterweise weist ein erfindungsgemäßes chirurgisches Instrument zwei gegensinnig verschwenkbare Werkzeuge auf, wobei das das eine Werkzeug verschwenkende Schwenkelement und der dieses verschwenkende Arm zu einem das andere Werkzeug verschwenkenden Schwenkelement beziehungsweise zu einem dieses verschwenkenden Arm symmetrisch bezüglich einer Drehung um 180° um die Schaftachse angeordnet sind. Beide Werkzeuge werden in gleicher Weise angetrieben, so daß die vorstehend und nachstehend gemachten Angaben zum Antrieb sich jeweils auf beide Werkzeuge beziehen.

Um Gegenstände ergreifen oder plastisch verformen zu können, ist es günstig, wenn das oder die Werkzeuge Zangenbacken aufweisen, die mit einer Zangenbacke eines gegensinnig verschwenkbaren Werkzeugs oder mit einer feststehenden Zangenbacke eine Zange bilden, die in der Öffnungsstellung des oder der Werkzeuge weiter geöffnet ist als in der Schließstellung.

Ist eine Mindestausdehnung der von der Zange zu greifenden oder plastisch zu verformenden Gegenstände vorgegeben, so ist es günstig, wenn die Zangenbacken in der Schließstellung um diese Mindestausdehnung voneinander beabstandet sind, damit die Schließstellung mit ihren günstigen Hebelverhältnissen beim bestimmungsgemäßen Gebrauch des chirurgischen Instruments auch tatsächlich erreicht werden kann.

Weitere Merkmale und Vorteile der Erfindung sind Gegenstand der nachfolgenden Beschreibung sowie der zeichnerischen Darstellung eines Ausführungsbeispiels.

### In der Zeichnung zeigen:

- Figur 1:: eine perspektivische Ansicht eines chirurgischen Instruments mit Werkzeugen in einer Öffnungsstellung;
- Figur 2:: einen Längsschnitt durch einen werkzeugseitigen Teil des chirurgischen Instruments längs Linie 2-2 in Figur 1;
- Figur 3:: einen vergrößerten Ausschnitt aus Figur 2;
- Figur 4:: eine Ansicht ähnlich Figur 3 mit Werkzeugen in einer Schließstellung;
- Figur 5:: einen Querschnitt durch den Schaft des chirurgischen Instruments längs Linie 5-5 in Figur 3 und
- Figur 6:: einen Längsschnitt durch einen Abschnitt des Schafts des chirurgischen Instruments längs Linie 6-6 in Figur 5.

Das in der Zeichnung dargestellte und als Ganzes mit 10 bezeichnete chirurgische Instrument umfaßt einen rohrförmigen Schaft 12, der an seinem hinteren Ende in eine einstückig angeformte und im wesentlichen quer abstehende Griffbranche 14 übergeht. Mit dieser ist gelenkig eine weitere Griffbranche 16 verbunden, die ihrerseits gelenkig mit einer Schub- und Zugstange 18 verbunden ist. Diese durchsetzt den Schaft 12 und ist im vorderen Teil des Schafts 12 mit einem in diesem längsverschieblichen, im wesentlichen zylindrischen Übertragungsglied 20 verbunden.

In ein vorderes Ende des Schafts 12 ist ein rohrförmiger Stutzen 22 eingeschraubt, der eine Verlängerung des Schafts 12 bildet und an seinem vorderen Ende zwei einander gegenüberliegende Lagerböcke 24 trägt. Diese Lagerböcke 24 bilden zwischen sich einen Zwischenraum 26 aus, in den die rückwärtigen Enden 28 von zwei gleich ausgebildeten Werkzeugen 30 eintauchen. Diese Werkzeuge 30 können beispielsweise an ihren vorderen Enden Zangenbacken 31 aufweisen, die zusammen eine Zange bilden. Sie sind in den Lagerböcken 24 durch einen diese durchsetzenden Stift 32 um eine Drehachse 34 verschwenkbar gelagert, die senkrecht zur Achse des rohrförmigen Stutzens 22 ausgerichtet ist.

Jedes der Werkzeuge 30 trägt an seinem rückwärtigen Ende 28 einen Schwenkhebel 36, der den rohrförmigen Stutzen 22 durchsetzt und in den Schaft 12 eintaucht.

Jeder Schwenkhebel 36 ist an einem ersten Anlenkpunkt 38 durch einen ersten Anlenkstift 39 gelenkig mit einem Arm 40 verbunden.

Die Senkrechten zur Drehachse 34 durch die ersten Anlenkpunkte 38 werden im folgenden als Schwenkhebelachsen 42 bezeichnet.

Jeder Arm 40 taucht in eine Ausnehmung 41 an einer vorderen Stirnseite des Übertragungsglieds 20 ein und ist an seinem freien Ende an einem zweiten Anlenkpunkt 44 durch einen zweiten Anlenkstift 45 gelenkig mit dem Übertragungsglied 20 verbunden.

Die durch den ersten Anlenkpunkt 38 und den zweiten Anlenkpunkt 44 jeden Arms 40 festgelegten Geraden werden im folgenden als Armachsen 46 bezeichnet.

Die beweglichen Teile des chirurgischen Instruments 10 weisen jeweils eine Öffnungsstellung, die mit einer Öffnungsstellung der Werkzeuge 30 korrespondiert, und eine Schließstellung, die mit einer Schließstellung der Werkzeuge 30 korrespondiert, auf.

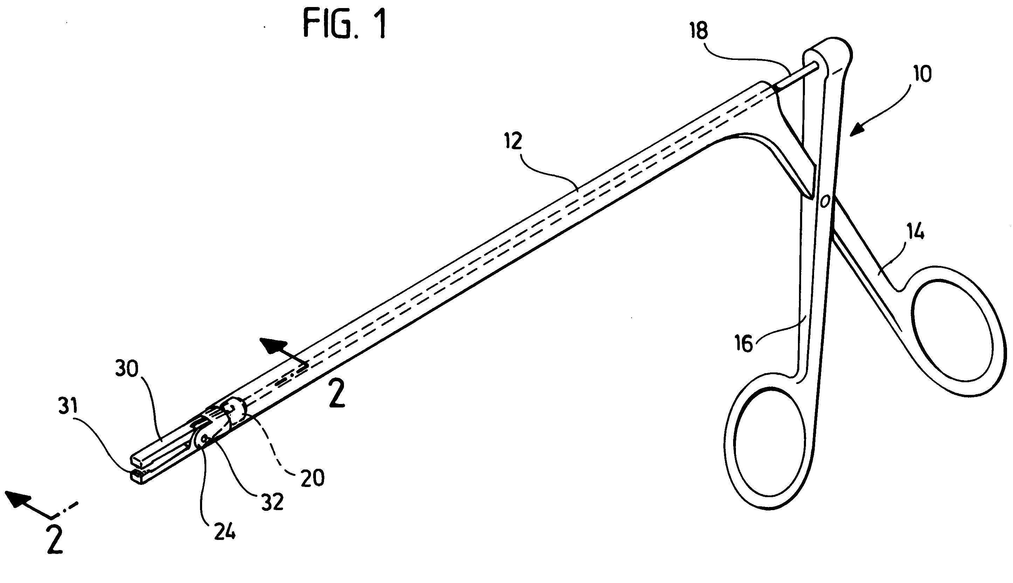

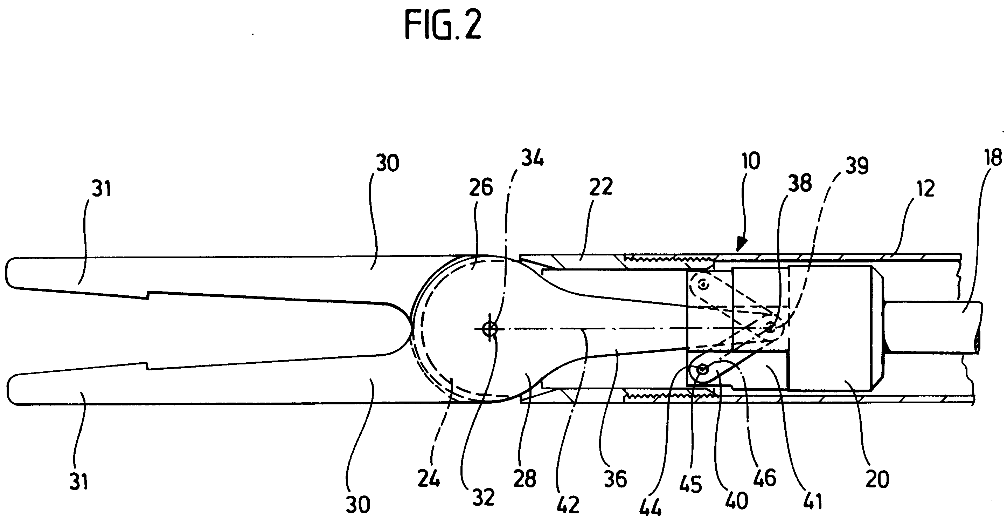

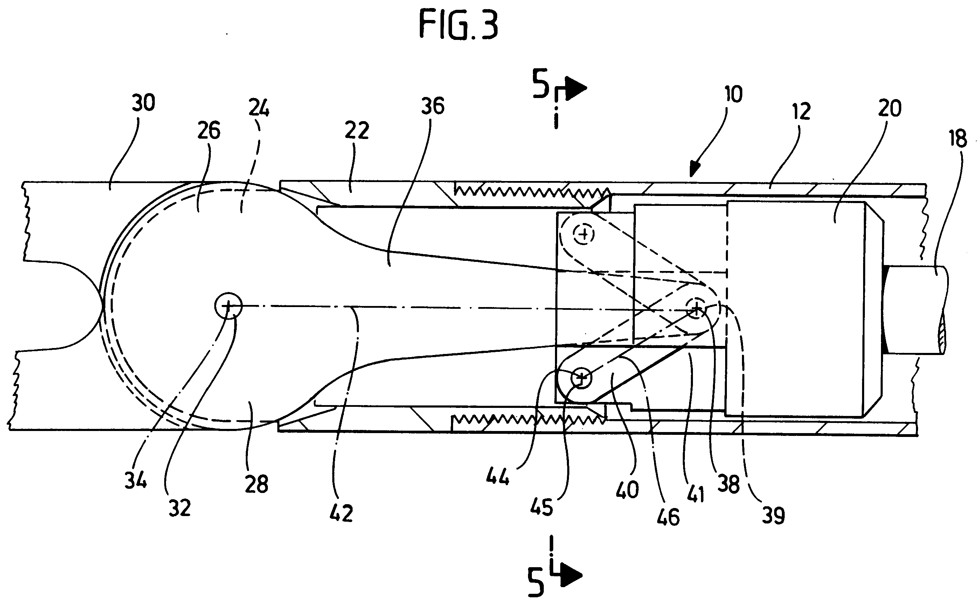

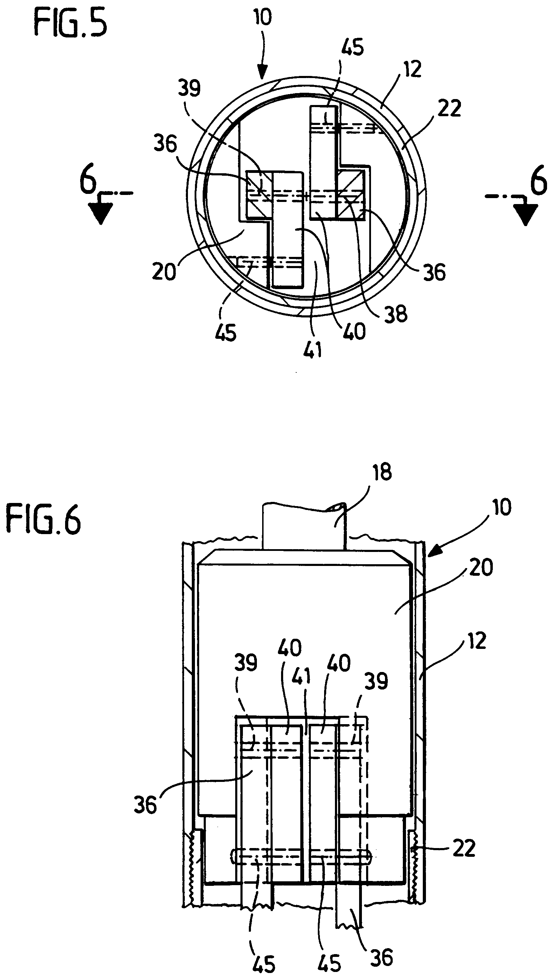

Die Öffnungsstellung ist in den Figuren 1 bis 3, 5 und 6 dargestellt.

In der Öffnungsstellung sind die Werkzeuge 30 um die Drehachse 34 maximal gegeneinander verdreht. Die von den Werkzeugen 30 mit den Zangenbacken 31 gebildete Zange ist in der Öffnungsstellung maximal geöffnet.

Die Schwenkhebel 36 sind in der Öffnungsstellung im wesentlichen längs der Schaftachse ausgerichtet, das heißt die Schwenkhebelachsen 42 verlaufen in geringem Abstand von der Schaftachse zu dieser parallel.

Jeder Arm 40 steht in der Öffnungsstellung von dem zugehörigen Schwenkhebel 36 schräg nach vorne hin ab, das heißt die Armachsen 46 bilden einen kleinen spitzen Winkel mit den Schwenkhebelachsen 42, und die zweiten Anlenkpunkte 44 sind, dem vorderen Ende des Schafts 12 näher als die ersten Anlenkpunkte 38, nahe der Innenwand des röhrenförmigen Stutzens angeordnet.

Aufgrund der spitzen Winkel zwischen den Armachsen 46 und den Schwenkhebelachsen 42 erzeugen durch die Arme 40 längs der Armachsen 46 auf die Schwenkhebel 36 übertragene Kräfte nur vergleichsweise kleine auf die Schwenkhebel 36 und auf die starr mit diesen verbundenen Werkzeuge 30 wirkende Drehmomente.

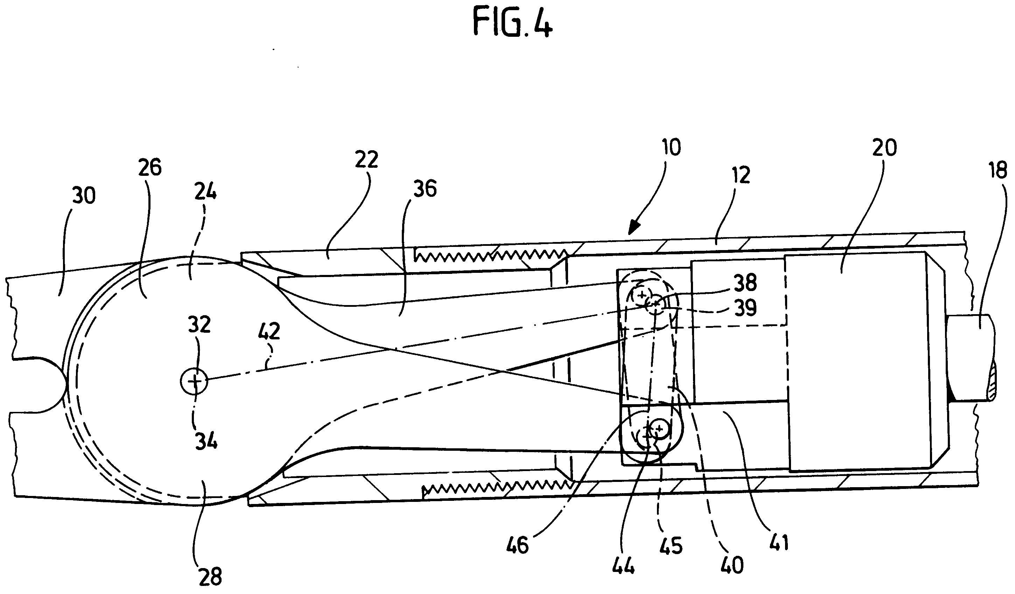

Durch Auseinanderspreizen der Griffbranchen 14 und 16 werden die beweglichen Teile des chirurgischen Instruments 10 von der vorstehend beschriebenen Öffnungsstellung in die in Figur 4 dargestellte Schließstellung gebracht.

Dabei wird das Übertragungsglied 20 von der Schub- und Zugstange 18 längs der Schaftachse nach hinten gezogen, wodurch auch die zweiten Anlenkpunkte 44 parallel zur Schaftachse nach hinten verschoben werden.

Die Arme 40 werden beim Übergang von der Öffnungs- zur Schließstellung (beim Schließen) um die zweiten Anlenkpunkte 44 verschwenkt, bis sie nahezu senkrecht zur Schaftachse ausgerichtet sind, das heißt bis die Armachsen 46 nahezu senkrecht zur Schaftachse stehen.

Die Schwenkhebel 36 und die starr mit diesen verbundenen Werkzeuge 30 werden dadurch ihrerseits um die Drehachse 34 verschwenkt, so daß sich die Zangenbacken 31 der Werkzeuge 31 aufeinanderzubewegen und sich die von den Zangenbacken 30 gebildete Zange schließt, bis die Zangenbacken 31 aneinander anliegen.

Da der radiale Abstand der ersten Anlenkpunkte 38 von der Drehachse 34 groß ist gegenüber der Länge der Arme 40, bewegen sich die ersten Anlenkpunkte 38 beim Übergang von der Öffnungs- in die Schließstellung im wesentlichen senkrecht zur Schaftachse von der Schaftmitte weg auf die Wand des Schafts 12 zu.

Dadurch, daß die Schließstellung erreicht wird, bevor die Arme 40 genau senkrecht zur Schaftachse ausgerichtet sind, wird verhindert, daß sich die Bewegungsrichtung der ersten Anlenkpunkte 38 beim Schließen umkehrt und sich die von den Zangenbacken 31 gebildete Zange wieder öffnet.

Wesentlich ist, daß beim Übergang von der Öffnungs- zur Schließstellung die Winkel zwischen den Armachsen 46 und den durch jeweils denselben Anlenkpunkt 38 verlaufenden Schwenkhebelachsen 42 sich immer mehr rechten Winkeln annähern, bis sie im wesentlichen 90° erreichen. Die Hebelverhältnisse für die Erzeugung von auf die Schwenkhebel 36 und damit die Werkzeuge 30 wirkenden Drehmomenten werden dadurch während des Schließens immer günstiger, das heißt, durch die Arme 40 längs der Armachse 46 auf die Schwenkhebel 36 übertragene Kräfte erzeugen während des Schließens wachsende Drehmomente, die in der Schließstellung selbst maximal sind.

Ein solcher Anstieg des Drehmoments während des Schließens ist besonders günstig, um einen zwischen den Zangenbacken 31 angeordneten Gegenstand, beispielsweise eine Operationsklammer aus Kunststoff oder Metall, während des Schließens plastisch zu verformen, weil auf diese Weise die während der Verformung wachsenden Gegendrehmomente leichter kompensiert werden können.

Ist eine Mindestausdehnung der zu ergreifenden Gegenstände oder der zu verformenden Gegenstände nach ihrer Verformung senkrecht zur Schaftachse vorgegeben, so werden die Werkzeuge 30 vorteilhafterweise so ausgestaltet, daß die Zangenbacken 31 in der Schließstellung um diese Mindestausdehnung voneinander beabstandet sind. Dadurch ist gewährleistet, daß die Schließstellung mit ihren günstigen Nebelverhältnissen beim Gebrauch des chirurgischen Instruments 10 auch tatsächlich genutzt werden kann.

Bei einer alternativen, nicht dargestellten Ausführungsform eines chirurgischen Instruments 10 stehen die Arme 40 in der Öffnungsstellung von den jeweiligen Schwenkhebeln 36 nicht in Schaftrichtung schräg nach vorne, sondern schräg nach hinten ab. Zum Übergang von der Öffnungs- in die Schließstellung werden in diesem Falle die Griffbranchen 14 und 16 aufeinanderzubewegt, so daß das mit der Schub- und Zugstange 18 verbundene Übertragungsglied 20 in Schaftrichtung nach vorne verschoben wird und die Arme 40 um die jeweiligen zweiten Anlenkpunkte 44 verschwenkt werden, bis sie nahezu senkrecht zur Schaftachse ausgerichtet sind. Im übrigen entspricht diese alternative Ausführungsform hinsichtlich Aufbau und Funktion der vorstehend beschriebenen.

## Patentansprüche

1. Chirurgisches Instrument (10), umfassend einen Schaft (12) mit einer Schaftachse, mindestens ein an dessen Ende verschwenkbar gelagertes Werkzeug (30), ein das Werkzeug (30) zwischen einer Öffnungs- und einer Schließstellung verschwenkendes, um eine Drehachse (34) verschwenkbar gelagertes Schwenkelement (36) und ein das Schwenkelement (36) um die Drehachse (34) verschwenkendes, von einem dem Werkzeug (30) gegenüberliegenden Ende des Schafts (12) aus betätigbares Betätigungsglied, das einen Arm (40) umfaßt, der an einem ersten Anlenkpunkt (38) gelenkig mit dem Schwenkelement (36) verbunden ist, wobei der Arm (40) in der Öffnungsstellung des Werkzeugs (30) schräg zu einer senkrecht zu der Drehachse (34) durch den ersten Anlenkpunkt (38) verlaufenden Achse (42) des Schwenkelements (36) und schräg zur Schaftachse und in der Schließstellung des Werkzeugs (30) im wesentlichen senkrecht zu der Achse (42) sowie nahezu senkrecht zur Schaftachse steht, dadurch gekennzeichnet, daß die Länge des Arms (40) im wesentlichen der lichten Weite des Schafts (12) entspricht.

2. Chirurgisches Instrument nach Anspruch 1, dadurch gekennzeichnet, daß der Arm (40) mit seinem freien Ende gelenkig mit einem längs der Schaftachse verschiebbaren Übertragungsglied (20) verbunden ist.

3. Chirurgisches Instrument nach einem der voranstehenden Ansprüche, dadurch gekennzeichnet, daß der radiale Abstand des ersten Anlenkpunkts (38) von der Drehachse (34) wesentlich größer ist als die lichte Weite des Schafts (12).

4. Chirurgisches Instrument nach einem der voranstehenden Ansprüche, dadurch gekennzeichnet, daß das Schwenkelement (36) starr mit dem Werkzeug (30) verbunden ist.

5. Chirurgisches Instrument nach einem der Ansprüche 1 bis 3, dadurch gekennzeichnet, daß das Schwenkelement (36) über Getriebemittel mit dem Werkzeug (30) verbunden ist.

6. Chirurgisches Instrument nach einem der voranstehenden Ansprüche, dadurch gekennzeichnet, daß das chirurgische Instrument (10) zwei gegensinnig verschwenkbare Werkzeuge (30) aufweist, wobei das das eine Werkzeug (30) verschwenkenden Schwenkelement (36) und der dieses verschwenkende Arm (40) zu dem das andere Werkzeug (30) verschwenkenden Schwenkelement (36) beziehungsweise zu dem dieses verschwenkenden Arm (40) symmetrisch bezüglich einer Drehung um 180° um die Schaftachse angeordnet sind.

7. Chirurgisches Instrument nach einem der voranstehenden Ansprüche, dadurch gekennzeichnet, daß das oder die Werkzeuge (30) Zangenbacken (31) aufweisen, die zusammen mit einer Zangenbacke (31) eines gegensinnig verschwenkbaren Werkzeugs (30) oder einer feststehenden Zangenbacke eine Zange bilden, die im Öffnungszustand des oder der Werkzeuge (30) weiter geöffnet ist als im Schließzustand des oder der Werkzeuge (30).

8. Chirurgisches Instrument nach Anspruch 7, dadurch gekennzeichnet, daß die die Zange bildenden Zangenbacken (31) im Schließzustand voneinander beabstandet sind.

## Claims

1. A surgical instrument (10) comprising a shaft (12) with a shaft axis, at least one implement (30) pivotably mounted on the end thereof, a pivoting member (36) mounted so as to pivot about a rotation axis (34) and pivoting the implement (30) between an open position and a closed position, and an actuating member pivoting the pivoting member (36) about the rotation axis (34), actuatable from the end of the shaft (12) opposite the implement (30) and comprising an arm (40) connected in an articulated manner to the pivoting member (36) at a first articulation point (38), wherein, in the open position of the implement (30), the arm (40) is arranged at an angle to an axis (42) of the pivoting member (36) extending through the first articulation point (38) perpendicularly to the rotation axis (34) and at an angle to the shaft axis, and, in the closed position of the implement (30), is arranged substantially perpendicularly to the axis (42) and substantially perpendicularly to the shaft axis, characterised in that the length of the arm (40) corresponds substantially to the inner diameter of the shaft (12).

2. A surgical instrument according to claim 1, characterised in that the free end of the arm (40) is connected in an articulated manner to a transmission member (20) displaceable along the shaft axis.

3. A surgical instrument according to either one of the preceding claims, characterised in that the radial distance of the first articulation point (38) from the rotation axis (34) is substantially greater than the inner diameter of the shaft (12).

4. A surgical instrument according to any one of the preceding claims, characterised in that the pivoting member (36) is rigidly connected to the implement (30).

5. A surgical instrument according to any one of claims 1 to 3, characterised in that the pivoting member (36) is connected to the implement (30) via transmission means.

6. A surgical instrument according to any one of the preceding claims, characterised in that the surgical instrument (10) has two implements (30) pivotable in opposite directions, the pivoting member (36) pivoting the one implement (30) and the arm (40) pivoting the said pivoting member (36) being arranged symmetrically to the pivoting member (36) pivoting the other implement (30) and the arm (40) pivoting the said pivoting member (36) with respect to rotation through 180° about the shaft axis.

7. A surgical instrument according to any one of the preceding claims, characterised in that the implement or implements (30) have forceps jaws (31) which, together with a forceps jaw (31) of an implement (30) pivotable in the opposite direction or a fixed forceps jaw, form forceps which are further open when the implement or implements (30) are open than when the implement or implements (30) are closed.

8. A surgical instrument according to claim 7, characterised in that the forceps jaws (31) forming the forceps are spaced apart when closed.

## Revendications

1. Instrument chirurgical (10) comprenant un fût (12) avec un axe de fût, au moins un outil (30) monté à pivotement sur son extrémité, un élément de pivotement (36) permettant de pivoter l'outil (30) entre une position d'ouverture et une position de fermeture, monté de manière à pouvoir pivoter autour d'un axe de rotation (34), et un organe d'actionnement faisant pivoter l'élément de pivotement (36) autour de l'axe de rotation (34), actionnable depuis l'extrémité du fût (12) opposée à l'outil (30), ledit organe comprenant un bras (40) relié en articulation avec l'élément de pivotement (36) sur un premier point d'articulation (38), l'arbre (40) se trouvant, dans la position d'ouverture de l'outil (30), en biais par rapport à un axe (42) de l'élément de pivotement (36), ledit axe s'étendant perpendiculairement à l'axe de rotation (34) à travers le premier point d'articulation (38), et en biais par rapport à l'axe du fût, et dans la position de fermeture de l'outil (30), ledit arbre se trouvant sensiblement perpendiculaire à l'axe (42) ainsi que presque perpendiculaire à l'axe du fût, caractérisé en ce que la longueur du bras (40) correspond sensiblement au diamètre intérieur du fût (12).

2. Instrument chirurgical selon la revendication 1, caractérisé en ce que le bras est relié de manière articulée par son extrémité libre avec un organe de transmission (20) déplaçable le long de l'axe du fût.

3. Instrument chirurgical selon l'une quelconque des revendications précédentes, caractérisé en ce que la distance radiale entre le premier point d'articulation (38) et l'axe de rotation (34) est sensiblement supérieure au diamètre intérieur du fût (12).

4. Instrument chirurgical selon l'une quelconque des revendications précédentes, caractérisé en ce que l'élément de pivotement (36) est relié de manière rigide à l'outil (30).

5. Instrument chirurgical selon l'une quelconque des revendications 1 à 3, caractérisé en ce que l'élément de pivotement (36) est relié à l'outil (30) par l'intermédiaire de moyens de transmission.

6. Instrument chirurgical selon l'une quelconque des revendications précédentes, caractérisé en ce que l'instrument chirurgical (10) présente deux outils (30) pivotables en sens inverse, l'élément de pivotement (36) faisant pivoter un premier outil (30) ainsi que le bras (40) faisant pivoter cet élément de pivotement étant agencés de manière symétrique par rapport à une rotation de 180° autour de l'axe du fût, par rapport à l'élément de pivotement (36) faisant pivoter l'autre outil (30) ou par rapport au bras (40) faisant pivoter cet élément de pivotement, respectivement.

7. Instrument chirurgical selon l'une quelconque des revendications précédentes, caractérisé en ce que le ou les outils (30) présentent des becs de pince (31) qui, conjointement avec un bec de pince d'un outil (30) susceptible d'être pivoté en sens inverse ou avec un bec de pince stationnaire, forment une pince qui à l'état d'ouverture de l'outil ou des outils (30) est ouverte plus largement qu'à l'état de fermeture de l'outil ou des outils (30).

8. Instrument chirurgical selon la revendication 7, caractérisé en ce que les becs (31) formant la pince sont espacés l'un de l'autre à l'état de fermeture.
